(19) Europäisches Patentamt / European Patent Office / Office européen des brevets

(11) **EP 4 610 190 A1**

(12) **EUROPEAN PATENT APPLICATION**
published in accordance with Art. 153(4) EPC

(43) Date of publication:
**03.09.2025 Bulletin 2025/36**

(21) Application number: **22963379.7**

(22) Date of filing: **24.10.2022**

(51) International Patent Classification (IPC):
***B65D 81/02*** *(2006.01)*   ***B65D 5/50*** *(2006.01)*

(52) Cooperative Patent Classification (CPC):
**B65D 5/50; B65D 81/02**

(86) International application number:
**PCT/JP2022/039445**

(87) International publication number:
**WO 2024/089727 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Japan Tobacco Inc.**
**Tokyo 105-6927 (JP)**

(72) Inventors:
• **NISHIGAI, Ken**
**Tokyo 130-8603 (JP)**
• **ASATO, Sho**
**Tokyo 130-8603 (JP)**

(74) Representative: **Hoffmann Eitle**
**Patent- und Rechtsanwälte PartmbB**
**Arabellastraße 30**
**81925 München (DE)**

(54) **TRAY FOR NON-COMBUSTIBLE FLAVOR INHALER**

(57)    This tray for a non-combustible flavor inhaler comprises a recessed part that is formed by folding a blank, and that is able to accommodate a non-combustible flavor inhaler, a hollow cushioning frame surrounding the recessed part, and a cushioning space formed in the interior of the cushioning frame, wherein the cushioning frame is formed so as to fit with and retain a non-combustible flavor inhaler accommodated in the recessed part.

[Fig. 1]

# Description

## TECHNICAL FIELD

The present disclosure relates to a tray for a heat-not-burn flavor inhaler.

## BACKGROUND ART

Conventionally, widespread use is made of packaging members for accommodating accommodated object such as electronic devices and for protecting the accommodated objects from impacts if they are dropped and vibration during transportation. A packing tray which is formed by folding a single sheet of cardboard multiple times, and which has an upwardly-opening accessory accommodating recess is known as a packaging member (e.g., PTL 1).

Furthermore, heat-not-burn flavor inhalers for allowing a flavor to be experienced without burning tobacco have become more popular in recent years in place of cigarettes. Heated cigarettes and electronic cigarettes are known as examples of such inhalers.

## CITATION LIST

## PATENT LITERATURE

   PTL 1 JP 2013-049434 A

   PTL 2 JP 2009-96520 A

   PTL 3 JP 3200836 U

   PTL 4 JP 2005-014965 A

## SUMMARY OF INVENTION

## TECHNICAL PROBLEM

A need for packaging members for protecting heat-not-burn flavor inhalers from impacts and vibration has arisen as heat-not-burn flavor inhalers have become more popular. However, conventional packaging members are not capable of adequately protecting heat-not-burn flavor inhalers.

The objective of the technology according to the present disclosure lies in providing a tray which is capable of suitably protecting a heat-not-burn flavor inhaler.

## SOLUTION TO PROBLEM

The technology according to the present disclosure employs the following features in order to solve the problem above. That is to say, the technology according to the present disclosure constitutes a heat-not-burn flavor inhaler tray formed by folding a blank and comprising: a recess capable of accommodating the heat-not-burn flavor inhaler; a hollow cushioning frame body surrounding the recess; and

a cushioning space formed inside the cushioning frame body, wherein the cushioning frame body is formed so as to fit with and hold the heat-not-burn flavor inhaler accommodated in the recess.

Furthermore, in the heat-not-burn flavor inhaler tray according to the present disclosure, an opening portion of the recess may be formed so as to follow an outer shape of the heat-not-burn flavor inhaler accommodated in the recess.

Furthermore, the heat-not-burn flavor inhaler tray may further comprise a bottom-face cushioning body forming a cushioning space on a bottom portion of the recess.

Furthermore, in the heat-not-burn flavor inhaler tray according to the present disclosure, the cushioning frame body may be formed by means of multiple hollow cushioning frame portions including: a first cushioning frame portion and a second cushioning frame portion extending in a first direction; and a third cushioning frame portion and a fourth cushioning frame portion extending in a second direction orthogonal to the first direction.

Furthermore, in the heat-not-burn flavor inhaler tray according to the present disclosure, both end portions of each of the first cushioning frame portion and the second cushioning frame portion may be inclined so that lengths thereof in the first direction decrease closer toward the recess in the second direction; both end portions of each of the third cushioning frame portion and the fourth cushioning frame portion may be inclined so that lengths thereof in the second direction decrease closer toward the recess in the first direction; one end portion of the third cushioning frame portion may be inserted at one end portion of the first cushioning frame portion so as to overlap said one end portion, whereby the third cushioning frame portion and the first cushioning frame portion may be joined; another end portion of the third cushioning frame portion may be inserted at one end portion of the second cushioning frame portion so as to overlap said one end portion, whereby the third cushioning frame portion and the second cushioning frame portion may be joined; one end portion of the fourth cushioning frame portion may be inserted at another end portion of the first cushioning frame portion so as to overlap said other end portion, whereby the fourth cushioning frame portion and the first cushioning frame portion may be joined; and another end portion of the fourth cushioning frame portion may be inserted at another end portion of the second cushioning frame portion so as to overlap said other end portion, whereby the fourth cushioning frame portion and the second cushioning frame portion may be joined.

Furthermore, in the heat-not-burn flavor inhaler tray according to the present disclosure, the pairs of cushioning frame portions of the cushioning frame body which are joined together may be configured so that, when θ1 is an angle of inclination in relation to the first direction of an end portion of a cushioning frame portion that receives an end portion of a counterpart side, and when θ2 is an angle of inclination in relation to the second direction of an end portion of a cushioning frame portion that is inserted at an end portion of a counterpart side, then: $30[°] \leq θ1 \leq 60[°]$, $55[°] \leq θ2 \leq 85[°]$, and $90[°] < θ1+θ2$.

Furthermore, in the heat-not-burn flavor inhaler tray according to the present disclosure, both end portions of each of the first cushioning frame portion and the second cushioning frame portion may be linearly inclined, and both end portions of each of the third cushioning frame portion and the second cushioning frame portion may be curved in an arc shape so as to expand outward.

Furthermore, in the heat-not-burn flavor inhaler tray according to the present disclosure, the heat-not-burn flavor inhaler may be sandwiched by a pair of cushioning frame portions of the cushioning frame body which face each other, and the pair of cushioning frame portions which face each other may be configured so that a gap therebetween becomes narrower from one side toward another side in a direction of extension of the pair of cushioning frame portions.

Furthermore, in the heat-not-burn flavor inhaler tray according to the present disclosure, the basis weight of the blank may be 400 [gsm] or greater.

Furthermore, in the heat-not-burn flavor inhaler tray according to the present disclosure, the blank may comprise: a square base panel having a first edge and a second edge extending in a first direction, and a third edge and a fourth edge extending in a second direction orthogonal to the first direction; and spacer panels joined to the edges of the base panel; and the cushioning frame body may be formed by folding the spacer panels.

Furthermore, in the heat-not-burn flavor inhaler tray according to the present disclosure, the cushioning frame body may be formed by multiple hollow cushioning frame portions; the blank may comprise: a first spacer panel joined to the first edge of the base panel, a second spacer panel joined to the second edge, a third spacer panel joined to the third edge, a fourth spacer panel joined to the fourth edge, and a cover panel joined to the fourth spacer panel; the first spacer panel, the second spacer panel, and the third spacer panel may each comprise: an outside tube wall panel which is joined to the base panel and folded so as to stand upright in relation to the base panel, a cover-side tube wall panel which is joined to the outside tube wall panel and folded so as to face the base panel, an inside tube wall panel which is joined to the cover-side tube wall panel and folded so as to face the outside tube wall panel, and a base-side tube wall panel which is joined to the inside tube wall panel and folded so as to face the cover-side tube wall panel and lies over the base panel, tubular cushioning frame portions being formed by folding the above panels; the fourth spacer panel may comprise: a base-side groove wall panel which is folded so as to lie over the base panel, a groove bottom wall panel which is joined to the base-side groove wall panel and folded so as to stand upright in relation to the base panel, and a cover-side groove wall panel which is joined to the groove bottom wall panel and folded so as to face the base-side groove wall panel, a recessed cushioning frame portion being formed by folding the above panels; the cover panel may be folded so as to face the base panel; and an opening of the recess may be formed in either one of the base panel and the cover panel.

Furthermore, in the heat-not-burn flavor inhaler tray according to the present disclosure, the opening of the recess may be formed in the base panel; the blank may further comprise a fifth spacer panel joined to any of the first spacer panel, the second spacer panel, the third spacer panel and the cover panel; and the fifth spacer panel may be folded so as to face the cover panel and thereby constitutes a bottom face of the recess while also forming a cushioning space with the cover panel.

ADVANTAGEOUS EFFECTS OF INVENTION

The technology according to the present disclosure lies makes it possible to suitably protect a heat-not-burn flavor inhaler.

BRIEF DESCRIPTION OF DRAWINGS

Fig. 1 is an oblique view of a heat-not-burn flavor inhaler tray according to Embodiment 1.

Fig. 2 is a front face view of the tray according to Embodiment 1.

Fig. 3 is an oblique view showing a state in which a heat-not-burn flavor inhaler is accommodated in the tray according to Embodiment 1.

Fig. 4 is a view in the cross section A-A in fig. 2.

Fig. 5 is a view in the cross section B-B in fig. 2.

Fig. 6 is a front face view of a cushioning frame body according to Embodiment 1.

Fig. 7 is a front face view showing a third cushioning frame portion.

Fig. 8 is a front face view showing a state in which an inhaler is accommodated in the tray according to Embodiment 1.

Fig. 9 is a plan view of a blank for forming the tray according to Embodiment 1.

Fig. 10 is a diagram (1) to illustrate a process of assembly of the tray according to Embodiment 1.

Fig. 11 is a diagram (2) to illustrate the process of assembly of the tray according to Embodiment 1.

Fig. 12 is an exploded diagram of a package constituting an example of a packaging box employing the tray according to Embodiment 1.

Fig. 13 is a rear face view of a front-side tray according to Embodiment 1.

Fig. 14 is an oblique view of a tray according to Embodiment 2.

Fig. 15 is a view in the cross section C-C in fig. 14.

Fig. 16 is a view in the cross section D-D in fig. 14.

Fig. 17 is a plan view of a blank for forming the tray according to Embodiment 2.

Fig. 18 is a diagram showing a step in the process of assembly of the tray according to Embodiment 2.

Fig. 19 is a table showing results of a drop test.

DESCRIPTION OF EMBODIMENTS

Embodiments according to the present disclosure will be described below with reference to the drawings. It should be noted that, unless specifically stated otherwise, materials, shapes and relative arrangements, etc. of the components disclosed in the following embodiments should not be construed as limiting the technical scope of the invention only to those mentioned. A heat-not-burn flavor inhaler, which is an object accommodated in the heat-not-burn flavor inhaler tray according to the present disclosure, is an inhaler for inhaling a flavor without associated burning. There is no particular limitation as to the specific structure, etc. of the heat-not-burn flavor inhaler according to the present disclosure, and it is possible to use a well-known inhaler, as appropriate. It should be noted that numerical value ranges indicated using "-" in the description mean ranges including the numerical values given before and after the "-" as a lower limit value and an upper limit value, for example, "A-B" means equal to or greater than A and equal to or less than B.

EMBODIMENT 1

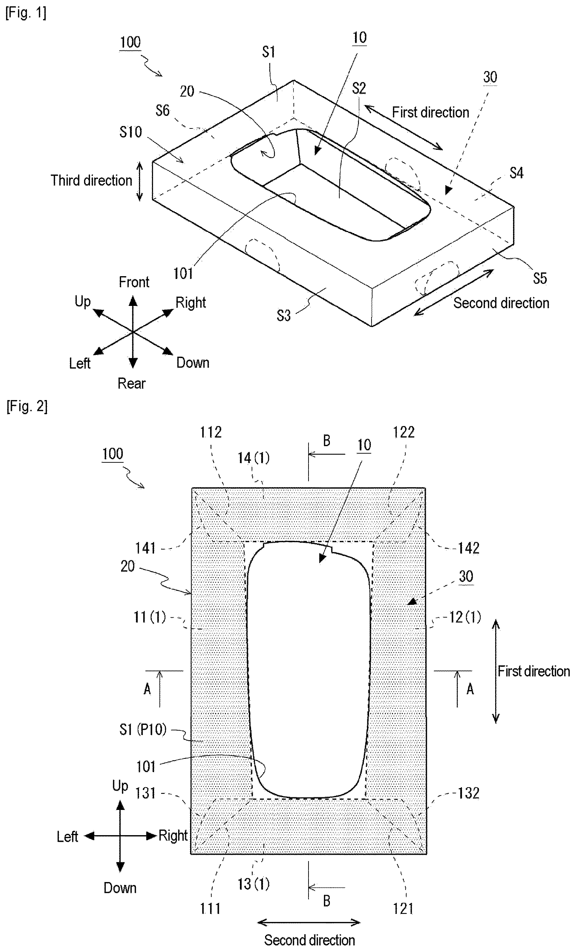

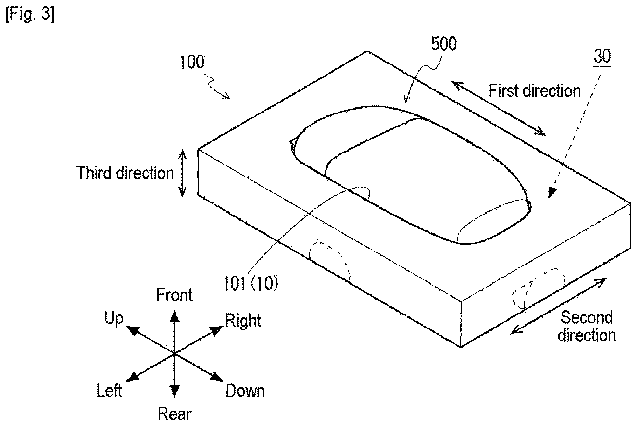

Fig. 1 is an oblique view of a heat-not-burn flavor inhaler tray 100 (referred to below as the "tray 100") according to Embodiment 1. Fig. 2 is a front face view of the tray 100 according to Embodiment 1. Fig. 3 is an oblique view showing a state in which a heat-not-burn flavor inhaler 500 (referred to below as the "inhaler 500") is accommodated in the tray 100 according to Embodiment 1. The arrows indicated in fig. 1-3 denote front/rear, up/down and left/right directions of the tray 100. It should be noted that, in Embodiment 1, the up/down direction corresponds to a "first direction", a left/right direction which is a direction orthogonal to the up/down direction corresponds to a "second direction", and a front/rear direction which is a direction orthogonal to the up/down direction and the left/right direction corresponds to a "third direction". However, the "first direction" and "second direction" according to the present disclosure are not limited to the up/down direction and the left/right direction. The second direction should be a direction orthogonal to the first direction.

Overall Configuration

As shown in fig. 3, the tray 100 according to Embodiment 1 is a packaging member for accommodating the inhaler 500. The inhaler 500 is a heating device for an electrically-heated inhaler (what is known as a heated cigarette). For example, the heating device heats a flavor inhalation article containing a tobacco material and an aerosol source to thereby cause an aerosol containing a flavoring component to be generated. However, the inhaler 500 is not limited to a heating device for an electrically-heated inhaler.

As shown in fig. 1, the tray 100 has an outer shape which is a substantially cuboid box shape. The tray 100 comprises: a front wall S1, a rear wall S2, a left side wall S3, a right side wall S4, a lower wall S5 and an upper wall S6. The front wall S1, rear wall S2, left side wall S3, right side wall S4, lower wall S5 and upper wall S6 constitute a front face, rear face, left side face, right side face, lower face and upper face, respectively, of the tray 100. The front wall S1 and rear wall S2 are orthogonal to the front/rear direction of the tray 100 and are also provided on opposite sides to each other in the front/rear direction. The left side wall S3 and right side wall S4 are orthogonal to the left/right direction of the tray 100 and are also provided on opposite sides to each other in the left/right direction. The lower wall S5 and the upper wall S6 are orthogonal to the up/down direction of the tray 100 and are also provided on opposite sides to each other in the up/down direction.

As shown in fig. 1 and 2, the tray 100 is provided with a recess 10 opening in the front wall S1. As shown in fig. 3, the tray 10 is configured to be capable of accommodating the inhaler 500 in the recess 10. Here, "accommodating" as used in the description means that at least part of the accommodated object is accommodated, and it is not necessary for the entire accommodated object to be accommodated. The recess 10 may therefore accommodate part of the inhaler 500 or accommodate the entire inhaler 500. As shown in fig. 3, roughly half of a rear side of the inhaler 500 is accommodated in the recess 10 of the tray 100 according to Embodiment 1.

Furthermore, as shown in fig. 1 and 2, the tray 100 comprises a cushioning frame body 20 formed in the shape of a frame so as to surround the recess 10. The cushioning frame body 20 is provided between the front wall S1 and the rear wall S2. In fig. 2, the recess 10 is indicated by a dot pattern. The recess 10 is formed by a space enclosed by the cushioning frame body 20. Furthermore, the cushioning frame body 20 is formed with a hollow shape inside which is formed a cushioning space 30 for protecting the inhaler 500 accommodated in the recess 10 from external impacts. The cushioning space 30 is formed along the entire periphery of the cushioning frame body 20. The inhaler 500 accommodated in the recess 10 is therefore enclosed by the cushioning space 20. This protects the inhaler 500 from external impacts on four sides (up/down/left/right).

As shown in fig. 2, the cushioning frame body 20 is formed by multiple hollow cushioning frame portions 1. Specifically, the cushioning frame body 20 includes: a first cushioning frame portion 11 and a second cushioning frame portion 12 extending in the up/down direction (first direction), and a third cushioning frame portion 13 and a fourth cushioning frame portion 14 extending in the left/-right direction (a second direction orthogonal to the first direction), and these cushioning frame portions are joined and thereby formed into a frame shape.

The first cushioning frame portion 11 and the second cushioning frame portion 12 face each other at an interval in the left/right direction, with the first cushioning frame portion 11 being disposed on the left side and the second cushioning frame portion 12 being disposed on the right side. The third cushioning frame portion 13 and the fourth cushioning frame portion 14 face each other at an interval in the up/down direction, with the third cushioning frame portion 13 being disposed on the lower side and the fourth cushioning frame portion 14 being disposed on the upper side.

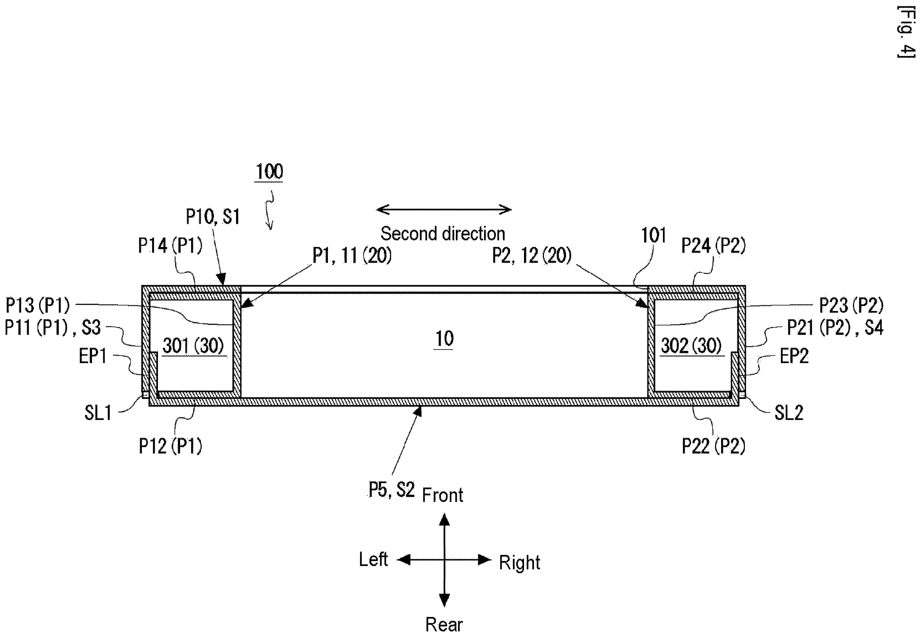

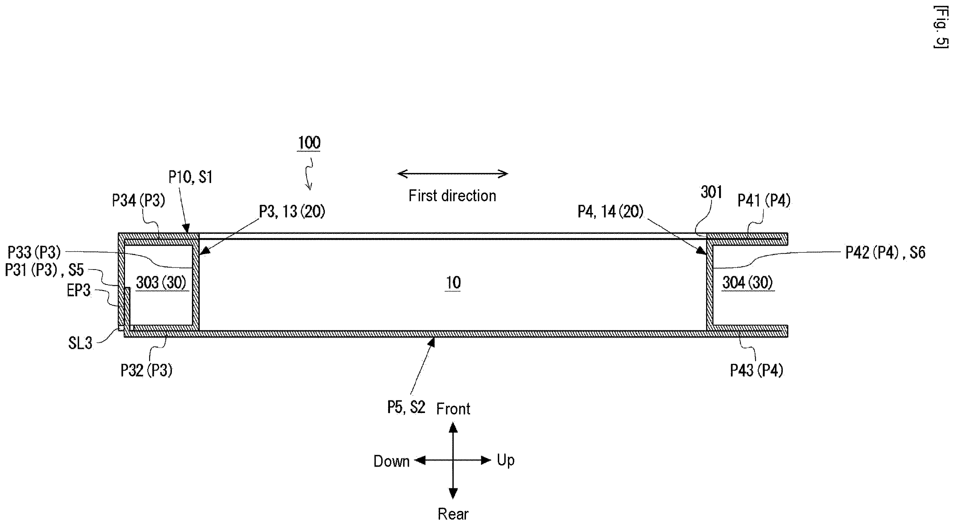

Fig. 4 is a view in the cross section A-A in fig. 2. Fig. 4 shows a cross section orthogonal to the first direction (up/down direction). Furthermore, fig. 5 is a view in the cross section B-B in fig. 2. Fig. 5 shows a cross section orthogonal to the second direction (left/right direction). As shown in fig. 4 and 5, the first cushioning frame portion 11, the second cushioning frame portion 12, and the third cushioning frame portion 13 are formed as tubular bodies with a square cross section. Furthermore, as shown in fig. 5, the fourth cushioning frame portion 14 is formed as a recessed body with a U-shaped cross section and is recessed in the first direction. A first space 301, a second space 302, a third space 303, and a fourth space 304 are formed inside the first cushioning frame portion 11, the second cushioning frame portion 12, the third cushioning frame portion 13, and the fourth cushioning frame portion 14, respectively. The first space 301, the second space 302, the third space 303, and the fourth space 304 are joined inside the cushioning frame body 20, and a frame-shaped cushioning space 30 is formed as a result.

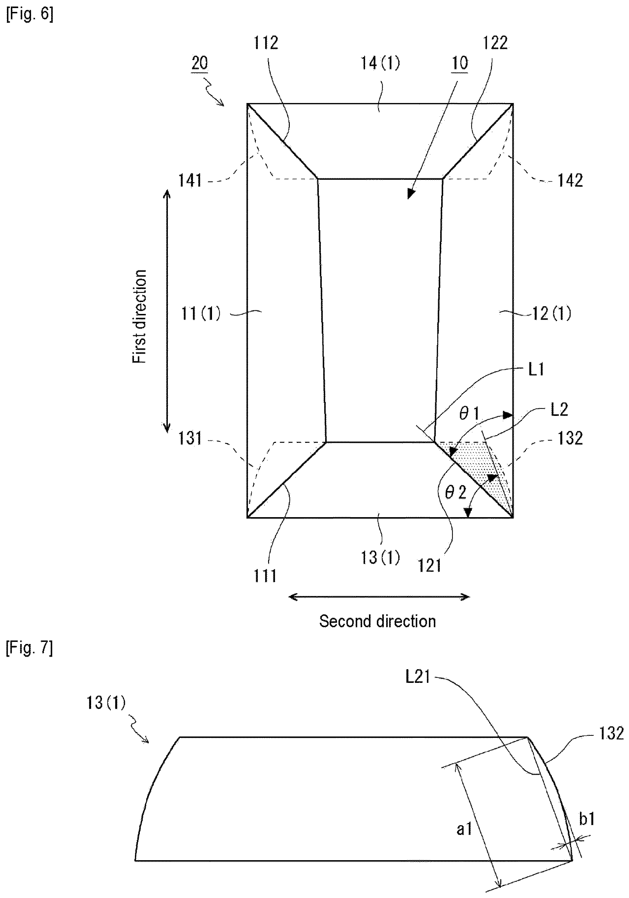

Here, fig. 6 is a front face view of the cushioning frame body 20 according to Embodiment 1. As shown in fig. 6, both end portions of each of the first cushioning frame portion 11 and the second cushioning frame portion 12 are inclined so that lengths thereof in the first direction (up/down direction) decrease closer toward the recess 10 in the second direction (left/right direction). Specifically, a lower end portion 111 of the first cushioning frame portion 11 is linearly inclined so as to displace upward toward the right side. Furthermore, an upper end portion 112 of the first cushioning frame portion 11 is linearly inclined so as to displace downward toward the right side. Similarly, a lower end portion 121 of the second cushioning frame portion 12 is linearly inclined so as to displace upward toward the left side. Furthermore, an upper end portion 122 of the second cushioning frame portion 12 is linearly inclined so as to displace downward toward the left side.

Furthermore, as shown in fig. 6, both end portions of each of the third cushioning frame portion 13 and the fourth cushioning frame portion 14 are inclined so that lengths thereof in the second direction (left/right direction) decrease closer toward the recess 10 in the first direction (up/down direction). Specifically, a left end portion 131 of the third cushioning frame portion 13 is inclined in a curved shape so as to displace to the right toward the upper side. Furthermore, a right end portion 132 of the third cushioning frame portion 13 is inclined in a curved shape so as to displace to the left toward the upper side. The left end portion 131 and the right end portion 132 of the third cushioning frame portion 13 are curved in an arc shape so as to expand outward (form outward projections). Similarly, a left end portion of the fourth cushioning frame portion 14 is inclined in a curved shape so as to displace to the right toward the lower side. Furthermore, a right end portion 142 of the fourth cushioning frame portion 14 is inclined in a curved shape so as to displace to the left toward the lower side. The left end portion 141 and the right end portion 142 of the fourth cushioning frame portion 14 are curved in an arc shape so as to expand outward.

As shown in fig. 6, the left end portion 131 (one end portion) of the third cushioning frame portion 13 is inserted at the lower end portion 111 (one end portion) of the first cushioning frame portion 11, whereby the third cushioning frame portion 13 and the first cushioning frame portion 11 are joined. Part of the left end portion 131 of the third cushioning frame portion 13 and part of the lower end portion 111 of the first cushioning frame portion 11 therefore overlap each other.

Furthermore, the right end portion 132 (another end portion) of the third cushioning frame portion 13 is inserted at the lower end portion 121 (one end portion) of the second cushioning frame portion 12, whereby the third cushioning frame portion 13 and the second cushioning frame portion 12 are joined. Part of the right end portion 132 of the third cushioning frame portion 13 and part of the lower end portion 121 of the second cushioning frame portion 12 therefore overlap each other.

Furthermore, the left end portion 141 (one end portion) of the fourth cushioning frame portion 14 is inserted at the upper end portion 112 (another end portion) of the first cushioning frame portion 11, whereby the fourth cushioning frame portion 14 and the first cushioning frame portion 11 are joined. Part of the left end portion 141 of the fourth cushioning frame portion 14 and part of the upper end portion 112 of the first cushioning frame portion 11 therefore overlap each other.

Furthermore, the right end portion 142 (another end portion) of the fourth cushioning frame portion 14 is inserted at the upper end portion 122 (another end portion) of the second cushioning frame portion 12, whereby the fourth cushioning frame portion 14 and the second cushioning frame portion 12 are joined. Part of the right end portion 142 of the fourth cushioning frame portion 14 and part of the upper end portion 122 of the second cushioning frame portion 12 therefore overlap each other.

The connections between the cushioning frame portions 1 of the cushioning frame body 20 will be described here. As described above, the first cushioning frame portion 11 and the third cushioning frame portion 13 of the cushioning frame body 20 are joined together, the first cushioning frame portion 11 and the fourth cushioning frame portion 14 are joined together, the second cushioning frame portion 12 and the third cushioning frame portion 13 are joined together, and the second cushioning frame portion 12 and the fourth cushioning frame portion 14 are joined together. The combinations of cushioning frame portions 1 of the cushioning frame body 20 which are joined together in the manner above will be referred to as the "pairs of cushioning frame portions which are joined together". Taking a combination of the second cushioning frame portion 12 and the third cushioning frame portion 13 as an example of a pair of cushioning frame portions 1, 1 which are joined together, the second cushioning frame portion 12 constitutes the cushioning frame portion 1 that receives the end portion (right end portion 132) of the counterpart side (third cushioning frame portion 13), and the third cushioning frame portion 13 constitutes the cushioning frame portion 1 that is inserted at the end portion (lower end portion 121) of the counterpart side (second cushioning frame portion 12).

Furthermore, the pairs of cushioning frame portions which are joined together are configured so that $\theta 1$ is an angle of inclination in relation to the first direction of an end portion of a cushioning frame portion 1 (i.e., the first cushioning frame portion 11 and the second cushioning frame portion 12) that receives an end portion of the counterpart side. That is to say, $\theta 1$ are the angles of inclination of the lower end portion 111 of the first cushioning frame portion 11, the upper end portion 112 of the first cushioning frame portion 11, the lower end portion 121 of the second cushioning frame portion 12, and the upper end portion 122 of the second cushioning frame portion 12. It should be noted that $\theta 1$ need not be the same and each $\theta 1$ may be different at the lower end portion 111 of the first cushioning frame portion 11, the upper end portion 112 of the first cushioning frame portion 11, the lower end portion 121 of the second cushioning frame portion 12, and the upper end portion 122 of the second cushioning frame portion 12. Here, the reference line L1 in fig. 6 denotes an imaginary straight line passing through both end points of an edge formed by the end portion of the receiving cushioning frame portion 1 as seen in a third direction orthogonal to the first direction and the second direction (seen in the front/rear direction in this example). As shown in fig. 6, $\theta 1$ is defined as the angle of inclination in relation to the first direction of the imaginary straight line L1.

Furthermore, the pairs of cushioning frame portions which are joined together are configured so that $\theta 2$ is an angle of inclination in relation to the second direction of an end portion of a cushioning frame portion 1 (i.e., the third cushioning frame portion 13 and the fourth cushioning frame portion 14) that is inserted at an end portion of the counterpart side. That is to say, $\theta 2$ are the angles of inclination of the left end portion 131 of the third cushioning frame portion 13, the right end portion 132 of the third cushioning frame portion 13, the left end portion 141 of the fourth cushioning frame portion 14, and the right end portion 142 of the fourth cushioning frame portion 14. It should be noted that $\theta 2$ need not be the same and each $\theta 2$ may be different at the right end portion 131 of the third cushioning frame portion 13, the left end portion 132 of the third cushioning frame portion 13, the left end portion 141 of the fourth cushioning frame portion 14, and the right end portion 142 of the fourth cushioning frame portion 14. As shown in fig. 6, $\theta 2$ is defined as the angle of inclination in relation to the second direction of an imaginary straight line L2 passing through both end points of an edge formed by the end portion of the inserted cushioning frame portion 1 as seen in the third direction.

In the tray 100 according to Embodiment 1, $\theta 1$ and $\theta 2$ are set so as to satisfy $30[°] \leq \theta 1 \leq 60[°]$, $55[°] \leq \theta 2 \leq 85[°]$, and $90[°] < \theta 1 + \theta 2$. However, the ranges of $\theta 1$ and $\theta 2$ are not limited to those given above in the technology according to the present disclosure.

By satisfying $90[°] < \theta 1 + \theta 2$, that is, by making the total of $\theta 1$ and $\theta 2$ greater than $90[°]$, the end portions of the pairs of cushioning frame portions which are joined together overlap. This makes it possible to ensure the strength of the tray 100. In addition, by setting $\theta 1$ and $\theta 2$ in the ranges of $30[°] \leq \theta 1 \leq 60[°]$ and $55[°] \leq \theta 2 \leq 85[°]$, ease of joining of the cushioning frame portions 1 is ensured, that is, ease of assembly of the tray 100 is ensured. It should be noted that $115[°] \leq \theta 1 + \theta 2$ is more preferred, and $\theta 1 + \theta 2 = 115[°]$ is most preferred, from the perspective of ensuring strength. In addition, $\theta 1 = 45[°]$ and $\theta 2 = 70[°]$ are most preferred from the perspective of ensuring strength.

Furthermore, in the tray 100, the pairs of cushioning frame portions which are joined together are configured so that the end portion of a cushioning frame portion 1 that is inserted at the end portion of the counterpart side (i.e., the third cushioning frame portion 13 and the fourth cushioning frame portion 14) is curved in an arc shape so as to expand outward. It is therefore possible to increase the area of overlap between the end portions of the pairs of cushioning frame portions which are joined together. This makes it possible to further increase the strength of the tray 100. Furthermore, by forming the end portions of cushioning frame portions 1 with an arc shape, it is possible to make those end portions easier to insert at the end portions on the counterpart side.

Fig. 7 is a front face view showing the third cushioning frame portion 13. The reference sign L21 in fig. 7 denotes an imaginary line segment connecting both end points of an edge formed by the end portion of the inserted cushioning frame portion 1 as seen in the third direction. As shown in fig. 7, a1 is the length of the imaginary line segment L21. Furthermore, b1 is the height of the end portion of the inserted cushioning frame portion 1 from the imaginary line segment L21 in a direction orthogonal to the imaginary line segment L21 and to the third direction. If the ratio [%] of b1 to a1 is c1, then $c_1 = b_1 \div a_1 \times 100$. In this case, from the perspective of a balance between improving strength and ease of assembly of the tray 100, the tray 100 preferably satisfies the conditions of: $55[°] \le \theta_2 \le 85[°]$ and $c_1[\%] = (85[°]-\theta_2[°]) \times 0.47[\%/°]$.

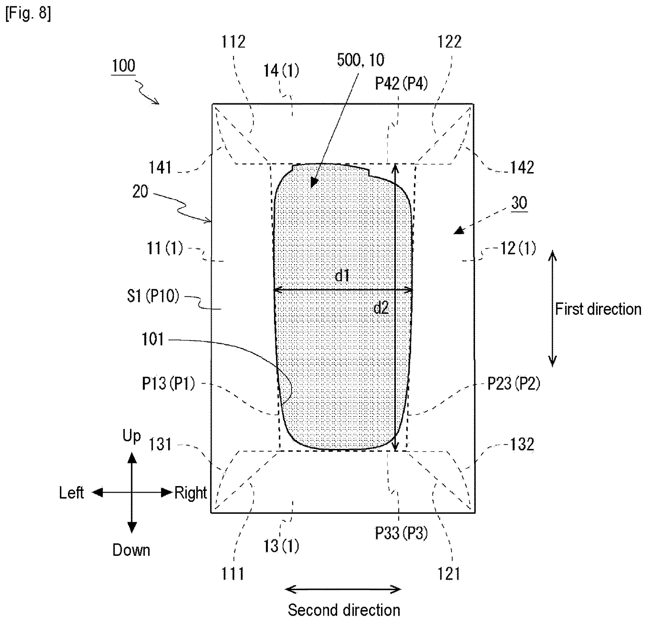

Fig. 8 is a front face view showing a state in which the inhaler 500 is accommodated in the tray 100 according to Embodiment 1. Here, as described above, the first cushioning frame portion 11 and the second cushioning frame portion 12 of the cushioning frame body 20 face each other, and the third cushioning frame portion 13 and the fourth cushioning frame portion 14 face each other. The combinations of cushioning frame portions 1 of the cushioning frame body 20 which face each other will be referred to as the "pairs of cushioning frame portions which face each other". The tray according to the present disclosure is configured so that the heat-not-burn flavor inhaler is sandwiched by at least one pair of cushioning frame portions of the cushioning frame body which face each other. As shown in fig. 8, in the tray 100 according to Embodiment 1, the inhaler 500 is sandwiched from the left/right direction (second direction) by the first cushioning frame portion 11 and second cushioning frame portion 12 which face each other, and the inhaler 500 is sandwiched from the up/down direction (first direction) by the third cushioning frame portion 13 and the fourth cushioning frame portion 14 which face each other. By this means, the inhaler 500 is fitted with and held by the cushioning frame body 20. It should be noted that the inhaler 500 may be sandwiched by only either one of the pair of cushioning frame portions 11, 12 and the pair of cushioning frame portions 13, 14.

The reference sign P13 shown in fig. 8 is a panel of the first cushioning frame portion 11 defining the recess 10 (that is, constituting the inner wall of the recess 10). Furthermore, the reference sign P23 is a panel of the second cushioning frame portion 12 defining the recess 10. Furthermore, the reference sign P33 is a panel of the third cushioning frame portion 13 defining the recess 10. Furthermore, the reference sign P42 is a panel of the fourth cushioning frame portion 14 defining the recess 10. As shown in fig. 7, the inside tube wall panel P13 and the inside tube wall panel P23 are not parallel to each other. Specifically, the inside tube wall panel P13 is inclined so as to displace to the right from the upper side toward the lower side. Furthermore, the inside tube wall panel P23 is inclined so as to displace to the left from the upper side toward the lower side. The pair of cushioning frame portions 11, 12 which face each other are configured so that a gap d1 therebetween becomes narrower from the upper side toward the lower side. Meanwhile, the inside tube wall panel P33 and the groove bottom wall panel P42 both extend along the up/down direction and are parallel to each other. As a result, a gap d2 between the pair of cushioning frame portions 13, 14 is constant in the left/right direction.

The gap between the pair of cushioning frame portions 11, 12 which face each other becomes narrower from one side (the upper side) toward another side (the lower side) in the first direction (up/down direction), which is the direction of extension of the pair of cushioning frame portions 11, 12, thereby making it possible for the pair of cushioning frame portions 11, 12 to securely sandwich the inhaler 500. This enables the inhaler 500 accommodated in the recess 10 to be stably held.

It should be noted that the pair of cushioning frame portions 11, 12 may also be configured so that the gap d1 therebetween becomes narrower from the lower side toward the upper side. Furthermore, the pair of cushioning frame portions 13, 14 which face each other may also be configured so that the gap d2 therebetween becomes narrower from one side toward another side in the direction of extension of the pair of cushioning frame portions 13, 14 (i.e., the second direction). The angles of the inside tube wall panel P13, the inside tube wall panel P23, the inside tube wall panel P33, and the groove bottom wall panel P42 may be appropriately set according to the shape of the inhaler 500 accommodated in the recess 10. In the tray 100, at least one pair of cushioning frame portions which face each other should be configured so that the gap therebetween becomes narrower from one side toward the other side in the direction of extension of that pair of cushioning frame portions. By so doing, the inhaler 500 can be stably held.

As shown in fig. 8, an opening portion 101 of the recess 10 is formed in the front wall S1. An opening edge of the opening portion 101 is formed so as to follow the outer shape of the inhaler 500 accommodated in the recess 10. It should be noted that the "outer shape of the inhaler 500" as referred to here more specifically denotes the outline of a cross section of the inhaler 500 when the inhaler 500 is cut on a plane orthogonal to the third direction (front/rear direction) at a position corresponding to the front wall S1 in the third direction. The opening edge of the recess 10 is formed so as to follow the outer shape of the inhaler 500, so a gap formed between the inhaler 500 and the cushioning frame body 20 when the inhaler 500 is accommodated in the recess 10 can be covered by the front wall S1. This makes it possible to produce a sense of fit between the inhaler 500 and the tray 100, regardless of the shape of the inhaler 500, and also makes it possible to improve the appearance.

The tray 100 holds the inhaler 500 by means of the cushioning frame body 20 in the manner described above, and the gap between the inhaler 500 and the cushioning frame body 20 is covered by the front wall S1, so the tray 100 is compatible with accommodating a variety of shapes.

Blank

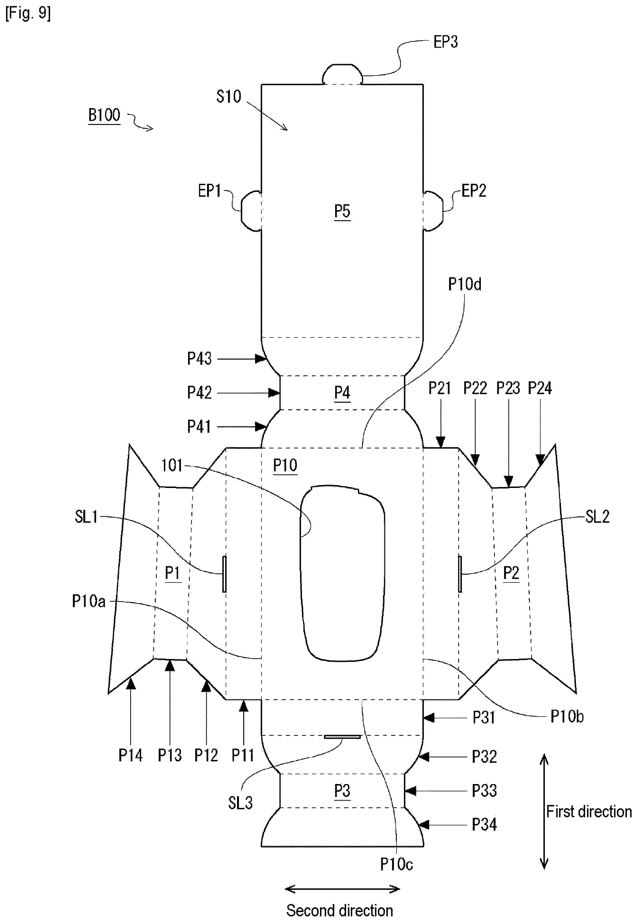

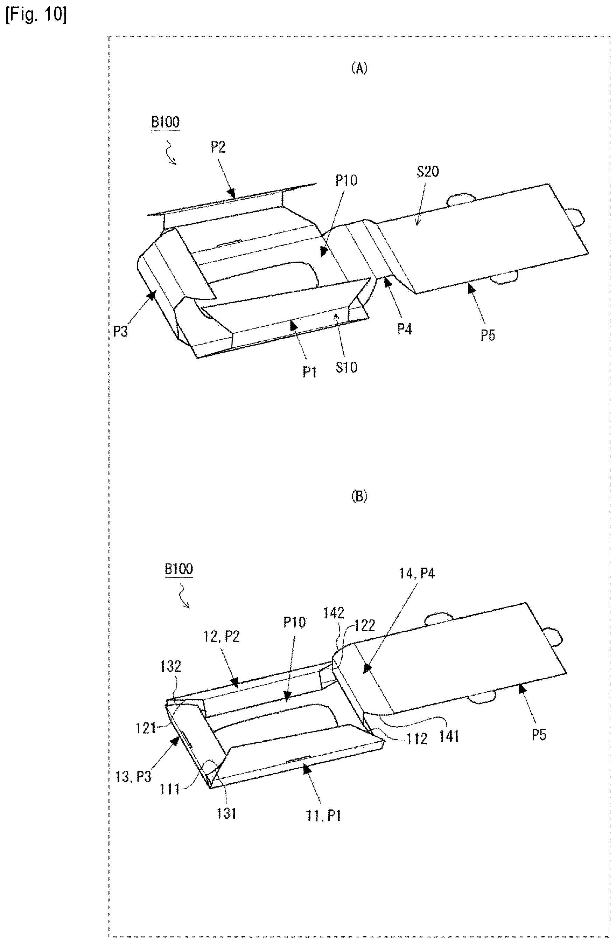

Fig. 9 is a plan view of a blank B100 for forming the tray 100 according to Embodiment 1. The blank B100 here has two faces: the face which can be seen in fig. 9 is a first face S10, and the face on the opposite side (rear side) is a second face S20 (see fig. 10(A)). The blank B100 is made of cardboard comprising pulp as the main material thereof, and is formed in the shape of a roughly rectangular sheet. The chain lines in fig. 9 denote fold lines. The tray 100 is assembled by folding one blank B100 at the boundaries of the fold lines. The blank B100 is shaped by punching the shape of the blank B100 from cardboard and forming fold lines or cut lines. There is no particular limitation as to the performance of the cardboard constituting the material of the blank B100, but the thickness of the blank B100 is preferably 400-600 [μm], from the perspective of protecting the inhaler 500, taking account of the weight of the inhaler 500. Furthermore, the basis weight of the blank B100 is preferably 400 [gsm] or greater, from the perspective indicated above. Furthermore, the basis weight of the blank B100 is preferably 600 gsm or less, from the perspectives of ease of shaping and production costs.

Cardboard is easily recycled so the environmental burden can be reduced by using cardboard for the tray 100, as compared to when a plastic material is used. The amount of water used in the production of the tray and the amount of $CO_2$ emitted can furthermore be reduced by using cardboard for the tray 100, as compared to when a pulp molding material is used. In addition, cardboard is easy to decorate with printing or the like, and using cardboard for the tray 100 therefore increases the degree of freedom in design. Furthermore, the tray 100 is assembled by folding the blank B100, so the development lead time, production lead time, and production costs can be reduced as compared to when the tray is produced by means of plastic molding or pulp molding which require a die to be made. It should be noted that the material of the blank according to the present disclosure is not limited to paper. It is sufficient for the blank to be formed by means of a bendable sheet material, and it may be formed by a plastic material or an aluminum material, for example.

As shown in fig. 9, the blank B100 comprises: a square base panel P10, and spacer panels P1, P2, P3, P4 joined to each edge of the base panel P10.

As shown in fig. 4, the base panel P10 is a panel constituting the front wall S1 of the tray 100. As shown in fig. 9, the base panel P10 comprises: a first edge P10a and a second edge P10b extending in the first direction (up/down direction); and a third edge P10c and a fourth edge P10d extending in the second direction (left/right direction). The first edge P10a and the second edge P10b have a relationship of opposite sides, with the first edge P10a constituting the left side of the base panel P10, and the second edge P10b constituting the right side of the base panel P10. The third edge P10c and the fourth edge P10d have a relationship of opposite sides, with the third edge P10c constituting the lower side of the base panel P10, and the fourth edge P10d constituting the upper side of the base panel P10. The opening portion 101 of the recess 10 is formed in the base panel P10.

The spacer panels P1, P2, P3, P4 are folded to thereby form the cushioning frame body 20. The first spacer panel P1 is joined to the first edge P10a of the base panel P10 and folded to thereby form the first cushioning frame portion 11. The second spacer panel P2 is joined to the second edge P10b of the base panel P10 and folded to thereby form the second cushioning frame portion 12. The third spacer panel P3 is joined to the third edge P10c of the base panel P10 and folded to thereby form the third cushioning frame portion 13. The fourth spacer panel P4 is joined to the fourth edge P10d of the base panel P10 and folded to thereby form the fourth cushioning frame portion 14.

As shown in fig. 9, the first spacer panel P1 comprises multiple panels P11-P14 joined in succession in the second direction by way of fold lines. Specifically, the first spacer panel P1 includes: an outside tube wall panel P11 joined to the base panel P10; a cover-side tube wall panel P12 joined to the outside tube wall panel P11; an inside tube wall panel P13 joined to the cover-side tube wall panel P12; and a base-side tube wall panel P14 joined to the inside tube wall panel P13. The base panel P10, the outside tube wall panel P11, the cover-side tube wall panel P12, the inside tube wall panel P13, and the base-side tube wall panel P14 are connected by way of fold lines. The first spacer panel P1 is folded at the boundaries of the fold lines to thereby form the tubular first cushioning frame portion 11. An insertion slit SL1 extending in the first direction is formed at the fold line connecting the outside tube wall panel P11 and the cover-side tube wall panel P12. As shown in fig. 4, the outside tube wall panel P11 is folded so as to stand upright in relation to the base panel P10 and thereby constitutes the left side wall S3 of the tray 100. The cover-side tube wall panel P12 is folded so as to face the base panel P10. The inside tube wall panel P13 is folded so as to face the outside tube wall panel P11. The base-side tube wall panel P14 is folded so as to face the cover-side tube wall panel P12, and is placed over the base panel P10.

As shown in fig. 9, the second spacer panel P2 comprises multiple panels P21-P24 joined in succession in the second direction by way of fold lines. Specifically, the second spacer panel P2 includes: an outside tube wall panel P21 joined to the base panel P10; a cover-side tube wall panel P22 joined to the outside tube wall panel P21; an inside tube wall panel P23 joined to the cover-side tube wall panel P22; and a base-side tube wall panel P24 joined to the inside tube wall panel P23. The base panel P10, the outside tube wall panel P21, the cover-side tube wall panel P22, the inside tube wall panel P23, and the base-side tube wall panel P24 are connected by way of fold lines. The second spacer panel P2 is folded at the boundaries of the fold lines to thereby form the tubular second cushioning frame portion 12. An insertion slit SL2 extending in the first direction is formed at the fold line connecting the outside tube wall panel P21 and the cover-side tube wall panel P22. As shown in fig. 4, the outside tube wall panel P21 is folded so as to stand upright in relation to the base panel P10 and thereby constitutes the right side wall S4 of the tray 100. The cover-side tube wall panel P22 is folded so as to face the base panel P10. The inside tube wall panel P23 is folded so as to face the outside tube wall panel P21. The base-side tube wall panel P24 is folded so as to face the cover-side tube wall panel P22, and is placed over the base panel P10.

As shown in fig. 9, the third spacer panel P3 comprises multiple panels P31-P34 joined in succession in the first direction by way of fold lines. Specifically, the third spacer panel P3 includes: an outside tube wall panel P31 joined to the base panel P10; a cover-side tube wall panel P32 joined to the outside tube wall panel P31; an inside tube wall panel P33 joined to the cover-side tube wall panel P32; and a base-side tube wall panel P34 joined to the inside tube wall panel P33. The base panel P10, the outside tube wall panel P31, the cover-side tube wall panel P32, the inside tube wall panel P33, and the base-side tube wall panel P34 are connected by way of fold lines. The third spacer panel P3 is folded at the boundaries of the fold lines to thereby form the tubular third cushioning frame portion 13. An insertion slit SL3 extending in the second direction is formed at the fold line connecting the outside tube wall panel P31 and the cover-side tube wall panel P32. As shown in fig. 5, the outside tube wall panel P31 is folded so as to stand upright in relation to the base panel P10 and thereby constitutes the lower side wall S5 of the tray 100. The cover-side tube wall panel P32 is folded so as to face the base panel P10. The inside tube wall panel P33 is folded so as to face the outside tube wall panel P31. The base-side tube wall panel P34 is folded so as to face the cover-side tube wall panel P32, and is placed over the base panel P10.

As shown in fig. 9, the fourth spacer panel P4 comprises multiple panels P41-P44 joined in succession in the first direction by way of fold lines. Specifically, the fourth spacer panel P4 includes: a base-side groove wall panel P41 joined to the base panel P10; a groove bottom wall panel P42 joined to the base-side groove wall panel P41; and a cover-side groove wall panel P43 joined to the groove bottom wall panel P42. The base panel P10, the base-side groove wall panel P41, the groove bottom wall panel P42, and the cover-side groove wall panel P43 are connected by way of fold lines. The fourth spacer panel P4 is folded at the boundaries of the fold lines to thereby form the recessed fourth cushioning frame portion 14. As shown in fig. 5, the base-side groove wall panel P41 is folded so as to overlap the base panel P10. The groove bottom wall panel P42 is folded so as to stand upright in relation to the base panel P10 and thereby constitutes the upper wall S6 of the tray 100. The cover-side groove wall panel P43 is folded so as to face the base-side groove wall panel P41.

As shown in fig. 9, the blank B100 further includes a square cover panel P5 which is adjacent to the fourth spacer panel P4 in the first direction and is joined to the cover-side groove wall panel P43 of the fourth spacer panel P4 by way of a fold line. The cover panel P5 is a panel constituting the rear wall S2 of the tray 100. As shown in fig. 9, the outer shape of the cover panel P5 is roughly the same as that of the first spacer panel P1. Insertion tabs EP1, EP2 are respectively provided on both side edges of the cover panel P5 in the second direction. Furthermore, an insertion tab EP3 is provided on an end portion of the cover panel P5 in the first direction. As shown in fig. 5, the cover panel P5 is folded so as to face the base panel P10 and thereby constitutes the rear wall S2 of the tray 100. As shown in fig. 4, the insertion tab EP1 is inserted into the insertion slit SL1 of the first spacer panel P1, and the insertion tab EP2 is inserted into the insertion slit SL2 of the second spacer panel P2. Furthermore, as shown in fig. 5, the insertion tab EP3 is inserted into the insertion slit SL3 of the third spacer panel P3. By this means, the cover panel P5 is fixed to the cushioning frame body 20 and the shape of the tray is retained. By using engagement of the insertion tabs and insertion slits, it is possible to retain the shape of the tray 100 without gluing or bonding using an adhesive tape.

As shown in fig. 4 and 5, the recess 10 is defined by: the inside tube wall panel P13 of the first spacer panel P1, the inside tube wall panel P23 of the second spacer panel P2, the inside tube wall panel P3 of the third spacer panel P3, the groove bottom wall panel P42 of the fourth spacer panel P4, and the cover panel P5. It should be noted that the opening portion 101 of the recess 10 may equally be formed in the cover panel P5 constituting the rear wall S2, rather than in the base panel P10 constituting the front wall S1 of the tray 100. The opening portion 101 of the recess 10 should be formed in either one of the base panel P10 and the cover panel P5.

Assembly Process

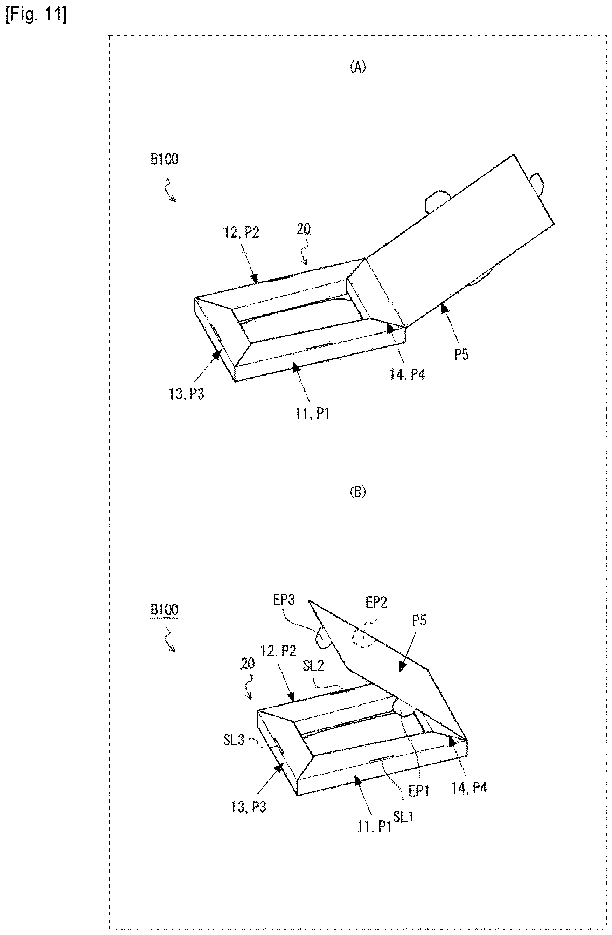

Fig. 10 and 11 are diagrams to illustrate the process of assembly of the tray 100 according to Embodiment 1. The process of assembly of the tray 100 will be described below with reference to fig. 10 and 11. First of all, as shown in fig. 10(A), the first spacer panel P1, the second spacer panel P2, the third spacer panel P3, and the fourth spacer panel P4 are folded in relation to the base panel P10 so that the first face S10 of the blank B100 faces outward, thereby forming the first cushioning frame portion 11, second cushioning frame portion 12, third cushioning frame portion 13, and fourth cushioning frame portion 14, as shown in fig. 10(B). As shown in fig. 10(B), the left end portion 131 of the third cushioning frame portion 13 is then inserted at the lower end portion 111 of the first cushioning frame portion 11, and the right end portion 132 is inserted at the lower end portion 121 of the second cushioning frame portion 12. Furthermore, the left end portion 141 of the fourth cushioning frame portion 14 is inserted at the upper end portion 112 of the first cushioning frame portion 11, and the right end portion 142 is inserted at the upper end portion 122 of the second cushioning frame portion 12. The frame-shaped cushioning frame body 20 is formed as a result, as shown in fig. 11(A). As shown in fig. 11(B), the cover panel P5 is then folded in relation to the fourth spacer panel P4 so that the cover panel P5 lies over the cushioning frame body 20, the insertion tab EP1 is inserted into the insertion slit SL1 of the first spacer panel P1, the insertion tab EP2 is inserted into the insertion slit SL2 of the second spacer panel P2, and the insertion tab EP3 is inserted into the insertion slit SL3 of the third spacer panel P3, whereby the cover panel P5 is fixed to the cushioning frame body 20. The tray 100 is assembled by this means. The tray 100 can be assembled in the above manner simply by folding the blank B100 without the need for gluing or bonding using an adhesive tape. Furthermore, the outer faces (front face, rear face, left side face, right side face, lower face, upper face) of the tray 100 are formed by the first face S10 of the blank B100. That is to say, the outer faces of the tray 100 are formed by only one face of the blank B100. The esthetic appearance of the tray 100 can therefore be improved simply by decorating one face (the first face S10 in this example) of the blank B100 with printing or the like, and this is also advantageous from a cost perspective.

Package

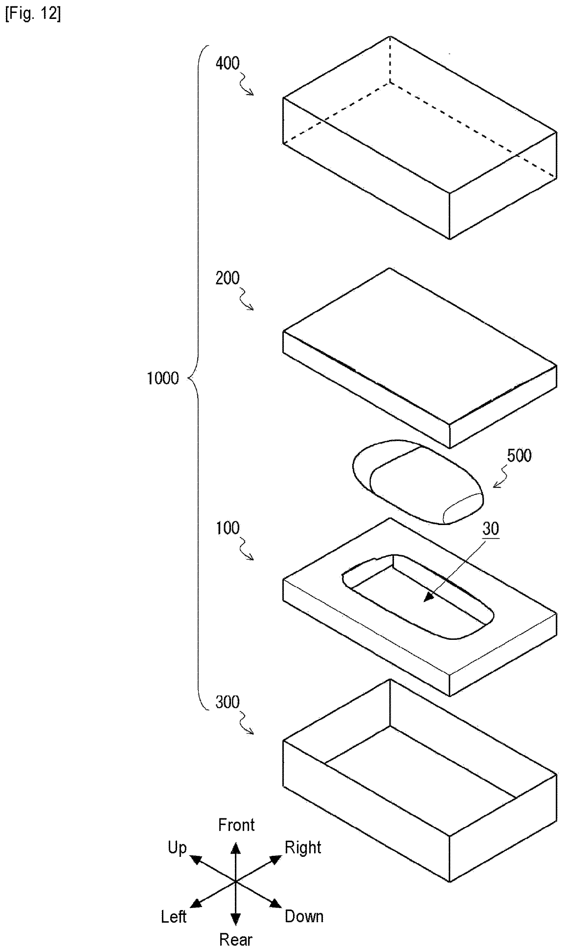

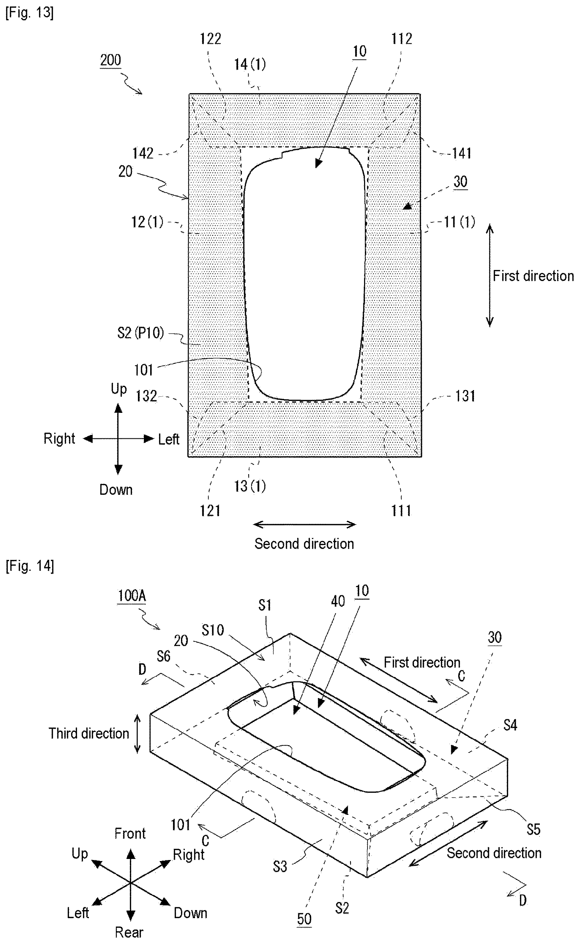

Fig. 12 is an exploded diagram of a package 1000 constituting an example of a packaging box employing the tray 100 according to Embodiment 1. As shown in fig. 12, the package 1000 according to Embodiment 1 is a box for packaging the inhaler 500, and comprises the tray 100, a front-side tray 200, a box bottom 300, and a box lid 400. The front-side tray 200 accommodates roughly half of the front side of the inhaler 500 from the opposite side to (i.e., the front side of) the tray 100. Fig. 13 is a rear face view of the front-side tray 200 according to Embodiment 1. As shown in fig. 13, the front-side tray 200 differs from the tray 100 in that a recess 10 opens in a rear wall S2 thereof so as to be capable of accommodating the inhaler 500 from the front side, but the front-side tray 200 is otherwise largely the same as the tray 100. As shown in fig. 12, the package 1000 sandwiches the inhaler 500 between the tray 100 and the front-side tray 200 from the front and rear, this assembly is then accommodated in the box bottom 300, and the resulting assembly is further covered by means of the box lid 400, whereby the inhaler 500 is packaged. In the package 1000, half of the rear side of the inhaler 500 is accommodated in the tray 100, and half of the front side of the inhaler 500 is accommodated in the front-side tray 200. The whole of the inhaler 500 is accommodated in the tray 100 and the front-side tray 200, whereby the inhaler 500 is suitably protected from impacts and vibration.

ACTION AND EFFECTS

As described above, the tray 100 according to Embodiment 1 is formed by folding the blank B100, and comprises: the recess 10 capable of accommodating the inhaler 500; the hollow cushioning frame body 20 surrounding the recess 10; and the cushioning space formed inside the cushioning frame body 20. The cushioning frame body 20 is then formed so as to fit with and hold the inhaler 500 accommodated in the recess 10. By virtue of this kind of tray 100, the inhaler 500 accommodated in the recess 10 is surrounded by the cushioning space 30 formed in a frame shape, and the inhaler 500 can therefore be suitably protected from impacts and vibration. Furthermore, the tray 100 is configured to fit with and hold the inhaler 500 by means of the cushioning frame body 20, and the inhaler 500 can therefore be securely gripped, even if it has a shape with curved portions and/or streamlined portions. Furthermore, the tray 100 comprises the hollow cushioning frame body 20, so the strength of the tray 100 can be ensured even if cardboard is used for the blank B100 rather than a material having a relatively large basis weight, such as corrugated fiberboard. Moreover, the tray 100 according to Embodiment 1 uses a smaller amount of the blank than a tray 100A according to Embodiment 2 which will be described below, to the extent that the tray 100 does not have a bottom-face cushioning body 40 (see fig. 14, etc.), so the tray 100 is superior to the tray 100A according to Embodiment 2 from an environmental perspective and a cost perspective.

In addition, the opening portion 101 of the recess 10 is formed so as to follow the outer shape of the inhaler 500 accommodated in the recess 10. As a result, a gap formed between the inhaler 500 and the cushioning frame body 20 when the inhaler 500 is accommodated in the recess 10 can be covered, and the appearance can be improved. Furthermore, the opening portion of the recess 10 has a shape which follows the outer shape of the inhaler 500, so the inhaler 500 can be gripped by the opening edge of the recess 10. This allows the inhaler 500 to be held more securely.

In addition, the cushioning frame body 20 is formed by means of multiple hollow cushioning frame portions 1 including: the first cushioning frame portion 11 and the second cushioning frame portion 12 extending in the first direction (up/down direction), and the third cushioning frame portion 13 and the fourth cushioning frame portion 14 extending in the second direction (left/right direction) orthogonal to the first direction. As a result, the inhaler 500 accommodated in the recess 10 can be surrounded from four sides by the cushioning space 30, and the inhaler 500 can therefore be more suitably protected.

In addition, both end portions of each of the first cushioning frame portion 11 and the second cushioning frame portion 12 are inclined so that lengths thereof in the first direction decrease closer toward the recess 10 in the second direction, and both end portions of each of the third cushioning frame portion 13 and the fourth cushioning frame portion 14 are inclined so that lengths thereof in the second direction decrease closer toward the recess 10 in the first direction. One end portion (the left end portion 131) of the third cushioning frame portion 13 is then inserted at one end portion (the lower end portion 111) of the first cushioning frame portion 11 so as to overlap said one end portion, whereby the third cushioning frame portion 13 and the first cushioning frame portion 11 are joined. Furthermore, another end portion (the right end portion 132) of the third cushioning frame portion 13 is inserted at one end portion (the lower end portion 121) of the second cushioning frame portion 12 so as to overlap said one end portion, whereby the third cushioning frame portion 13 and the second cushioning frame portion 12 are joined. Furthermore, one end portion (the left end portion 141) of the fourth cushioning frame portion 14 is inserted at another end portion (the upper end portion 112) of the first cushioning frame portion 11 so as to overlap said other end portion, whereby the fourth cushioning frame portion 14 and the first cushioning frame portion 11 are joined. Furthermore, another end portion (the right end portion 142) of the fourth cushioning frame portion 14 is inserted at another end portion (the upper end portion 122) of the second cushioning frame portion 12 so as to overlap said other end portion, whereby the fourth cushioning frame portion 14 and the second cushioning frame portion 12 are joined. In this way, the cushioning frame portions 1 are joined with overlapping end portions, thereby making it possible to ensure the strength of the tray 100.

Furthermore, in the tray 100 according to Embodiment 1, the pairs of cushioning frame portions of the cushioning frame body 20 which are joined together are configured so that, when $\theta 1$ is the angle of inclination in relation to the first direction of an end portion of a cushioning frame portion that receives an end portion of a counterpart side, and when $\theta 2$ is the angle of inclination in relation to the second direction of an end portion of a cushioning frame portion that is inserted at an end portion of a counterpart side, then $\theta 1$ and $\theta 2$ are set so that: $30[°] \leq \theta 1 \leq 60[°]$, $55[°] \leq \theta 2 \leq 85[°]$, and $90[°] < \theta 1 + \theta 2$. This makes it possible to ensure the strength and ease of assembly of the tray 100.

In addition, both end portions of each of the first cushioning frame portion 11 and the second cushioning frame portion 12 are linearly inclined, and both end portions of each of the third cushioning frame portion 13 and the fourth cushioning frame portion 14 are curved in an arc shape so as to expand outward. As a result, it is possible to increase the area of overlap between the end portions of the pairs of cushioning frame portions which are joined together, and the strength of the tray can be further increased. Furthermore, by forming the end portions of cushioning frame portions 1 with an arc shape, it is possible to make those end portions easier to insert at the end portions on the counterpart side.

In addition, in the tray 100 according to Embodiment 1, the inhaler 500 is sandwiched by a pair of cushioning frame portions 1, 1 which face each other among the plurality of cushioning frame portions 1, and the pair of cushioning frame portions 1, 1 which face each other are configured so that the gap therebetween becomes narrower from one side toward another side in the direction of extension of the pair of cushioning frame portions 1, 1. In this way, the inhaler 500 can be securely sandwiched by the pair of cushioning frame portions 1, 1 which face each other, and the inhaler 500 can be stably held.

Furthermore, the blank B100 according to Embodiment 1 comprises: the square base panel P10 having the first edge P10a and the second edge P10b extending in the first direction, and the third edge P10c and the fourth edge P10d extending in the second direction; and the spacer panels P1, P2, P3, P4 joined to the edges of the base panel P10. The spacer panels P1, P2, P3, P4 are then folded to thereby form the cushioning frame body 20. By using such a blank B100, the tray 100 can be formed by a single blank.

More specifically, the blank B100 according to Embodiment 1 includes: the first spacer panel P1 joined to the first edge P10a of the base panel P10; the second spacer panel P2 joined to the second edge P10b; the third spacer panel P3 joined to the third edge P10c; the fourth spacer panel P4 joined to the fourth edge P10d; and the cover panel P5 joined to the fourth spacer panel P4. The first spacer panel P1, the second spacer panel P2, and the third spacer panel P3 then respectively comprise: the outside tube wall panels P11, P21, P31 which are joined to the base panel P10 and folded so as to stand upright in relation to the base panel P10; the cover-side tube wall panels P12, P22, P32 which are joined to the outside tube wall panels P11, P21, P31 and folded so as to face the base panel P10; the inside tube wall panels P13, P23, P33 which are joined to the cover-side tube wall panels P12, P22, P32 and folded so as to face the outside tube wall panels P11, P21, P31, and the base-side tube wall panels P14, P24, P34 which are joined to the inside tube wall panels P13, P23, P33 and folded so as to face the cover-side tube wall panels P12, P22, P32 and lie over the base panel P10, the tubular cushioning frame portions 11, 12, 13 being formed by folding the above panels. Furthermore, the fourth spacer panel P4 comprises: the base-side groove wall panel P41 which is folded so as to lie over the base panel P10, the groove bottom wall panel P42 which is joined to the base-side groove wall panel P41 and folded so as to stand upright in relation to the base panel P10, and the cover-side groove wall panel P43 which is joined to the groove bottom wall panel P42 and folded so as to face the base-side groove wall panel P41, the recessed cushioning frame portion 14 being formed by folding the above panels. The cover panel P5 is folded so as to face the base panel P10. The opening portion 101 of the recess 10 is then formed in either one of the base panel P10 and the cover panel P5. By using such a blank B100, the cushioning frame body 20 can be formed into a frame shape.

EMBODIMENT 2

A heat-not-burn flavor inhaler tray 100A (referred to below as the "tray 100A" according to Embodiment 2 will be described below. The description of Embodiment 2 will focus on differences with Embodiment 1, and components which are the same as those of Embodiment 1 will be assigned the same reference signs to avoid a detailed description thereof.

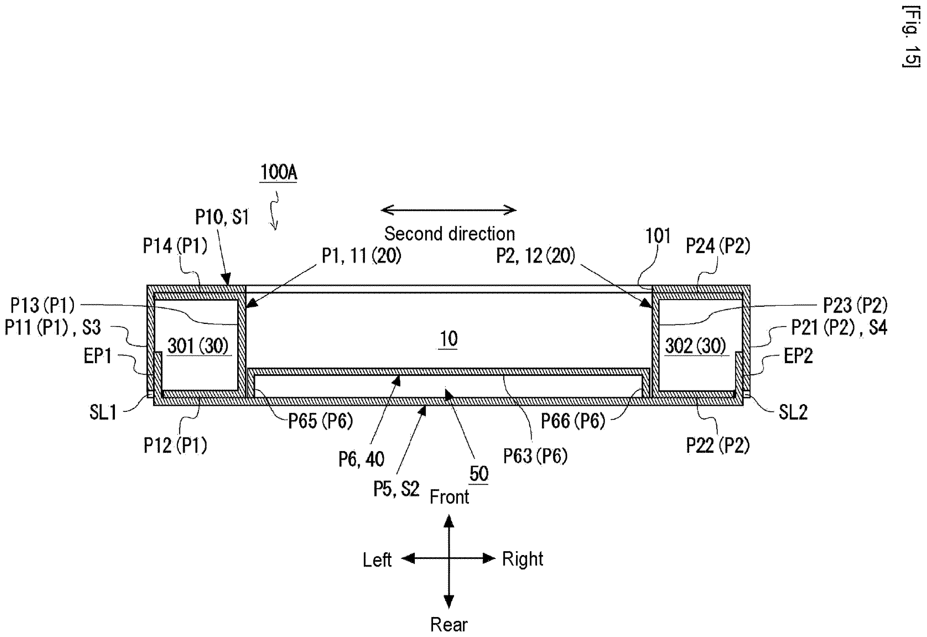

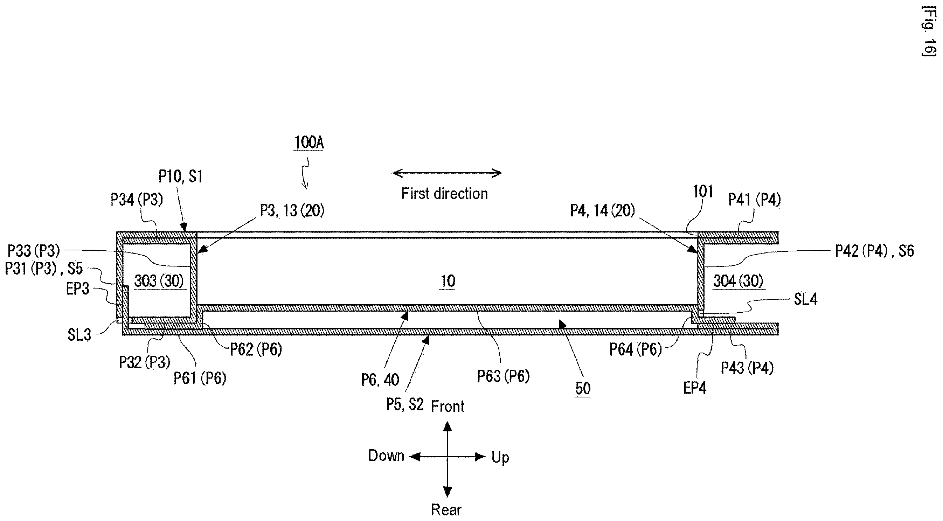

Fig. 14 is an oblique view of the tray 100A according to Embodiment 2. Furthermore, fig. 15 is a view in the cross section C-C in fig. 14. Fig. 15 shows a cross section orthogonal to the first direction (up/down direction). Furthermore, fig. 16 is a view in the cross section D-D in fig. 14. Fig. 16 shows a cross section orthogonal to the second direction (left/right direction).

As shown in fig. 14-16, the tray 100A according to Embodiment 2 differs from the tray 100 according to Embodiment 1 in that the tray 100A comprises a bottom-face cushioning body 40. The bottom-face cushioning body 40 forms a cushioning space 50 at the bottom portion of the recess 10. More specifically, the bottom-face cushioning body 40 constitutes the bottom face of the recess 10 while also forming the cushioning space 50 with the rear wall S2 (cover panel P5) of the tray 100A.

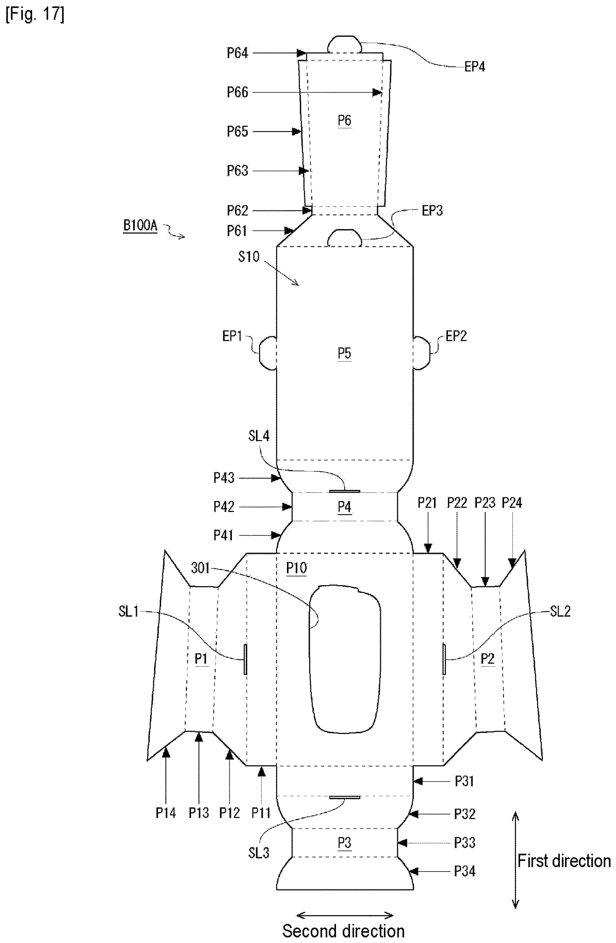

Fig. 17 is a plan view of a blank B100A for forming the tray 100A according to Embodiment 2. As shown in fig. 14-17, the blank B100A according to Embodiment 2 differs from the blank B100 according to Embodiment 1 in that the blank B100A comprises a fifth spacer panel P6 joined to the cover panel P5. The fifth spacer panel P6 is a panel constituting the bottom-face cushioning body 40 of the tray 100A, is adjacent to the cover panel P5 in the first direction, and is joined to the cover panel P5 by way of a fold line. Moreover, the opening portion 101 of the recess 10 is formed in the base panel P10, in the same way as in Embodiment 1.

As shown in fig. 17, the fifth spacer panel P6 includes multiple panels P61-P66. The panels P61-P64 are joined in succession in the first direction by way of fold lines. The panels P65, P66 are joined to both side edges of the panel P63 in the second direction. Furthermore, an insertion tab EP4 is provided on an end portion of the fifth spacer panel P6 in the first direction.

An insertion slit SL4 extending in the second direction is formed at the fold line connecting the groove bottom wall panel P42 and the cover-side groove wall panel P43 of the fourth spacer panel P4. Furthermore, in the blank B100A, the insertion tab EP3 is formed by part of the panel P61.

It should be noted that it is not essential for the fifth spacer panel P6 to be joined to the cover panel P5, and it is equally possible for the fifth spacer panel P6 to be joined to any of the first spacer panel P1, the second spacer panel P2, the third spacer panel P3, and the cover panel P5.

As shown in fig. 15 and 16, the fifth spacer panel P6 is folded so as to face the cover panel P5 and thereby constitutes the bottom face of the recess 10 while also forming the cushioning space 50 with the cover panel P5. Specifically, as shown in fig. 16, the panel P61 is folded so as to be interposed between the cover panel P5 and the cover-side tube wall panel P32 of the third spacer panel P3. The panel P62 is folded so as to stand upright in relation to the cover panel P5 along the inside tube wall panel P33 of the third spacer panel P3. The panel P63 is folded so as to face the cover panel P5 at an interval from the cover panel P5. The panel P64 is folded so as to stand upright in relation to the cover panel P5 along the groove bottom wall panel P42 of the fourth spacer panel P4. Furthermore, as shown in fig. 15, the panel P65 is folded so as to stand upright in relation to the cover panel P5 along the inside tube wall panel P13 of the first spacer panel P1. The panel P66 is folded so as to stand upright in relation to the cover panel P5 along the inside tube wall panel P23 of the second spacer panel P2.

As shown in fig. 15 and 16, the bottom face of the recess 10 is formed by the panel P63. Furthermore, the cushioning space 50 is formed as a result of the panel P63 being formed at an interval from the cover panel P5. Furthermore, as shown in fig. 16, the insertion tab EP4 is inserted into the insertion slit SL4 of the fourth spacer panel P4. By this means, the fifth spacer panel P6 is fixed to the cushioning frame body 20.

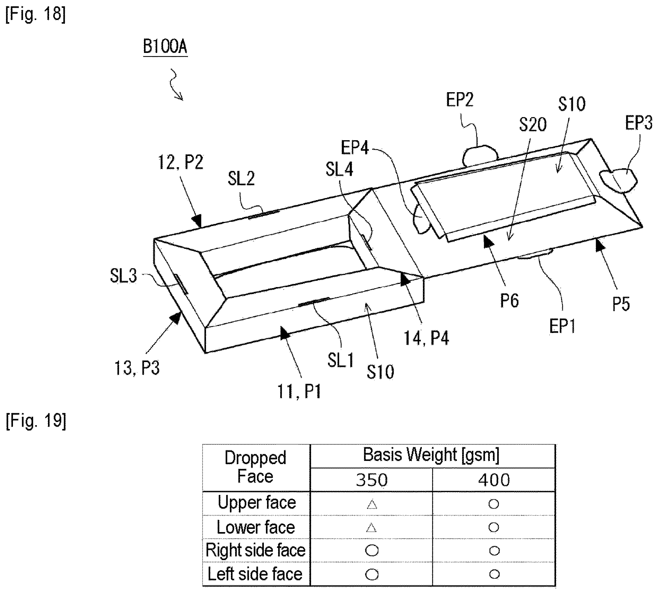

Fig. 18 is a diagram showing a step in the process of assembly of the tray 100A according to Embodiment 2. As shown in fig. 18, in a state in which the cushioning frame body 20 has been formed by folding the spacer panels P1-P4, the cover panel P5 is folded in relation to the fourth spacer panel P4 so that the cover panel P5 lies over the cushioning frame body 20, and the insertion tab EP4 is inserted into the insertion slit SL4 while folding the fifth spacer panel P6, whereby the bottom-face cushioning body 40 is formed. The insertion tab EP1 is then inserted into the insertion slit SL1 of the first spacer panel P1 , the insertion tab EP2 is inserted into the insertion slit SL2 of the second spacer panel P2, and the insertion tab EP3 is inserted into the insertion slit SL3 of the third spacer panel P3, whereby the cover panel P5 is fixed to the cushioning frame body 20. The tray 100A is assembled by this means.

ACTION AND EFFECTS

The tray 100A according to Embodiment 2 also makes it possible to achieve the same action and effects as the tray 100 according to Embodiment 1. That is to say, by virtue of the tray 100A, the inhaler 500 is surrounded by the frame-shaped cushioning space 30, and the inhaler 500 can therefore be suitably protected from impacts and vibration.

The tray 100A according to Embodiment 2 then further comprises the bottom-face cushioning body 40 which forms the cushioning space 50 on the bottom portion of the recess 10. As a result, not only is the inhaler 500 protected by the cushioning space 30 from the side-face sides of the recess 10, the inhaler 500 can also be protected by the cushioning space 50 from the bottom portion side of the recess 10. As a result, the inhaler 500 can be more suitably protected from impacts and vibration.

Furthermore, the blank B100A according to Embodiment 2 comprises the fifth spacer panel P6 joined to any of the first spacer panel P1, the second spacer panel P2, the third spacer panel P3 and the cover panel P5, and the fifth spacer panel P6 is folded so as to face the cover panel P5 and thereby constitutes the bottom face of the recess 10 while also forming the cushioning space 50 with the cover panel P5. By using such a blank B100A, the tray 100A can be formed by a single blank. It should be noted that, in Embodiment 2, in addition to the outer faces (front face, rear face, left side face, right side face, lower face, upper face) of the tray 100, the bottom face of the recess 10 is also formed by the first face S10 of the blank B100A. That is to say, the outer faces of the tray 100 and the bottom face of the recess 10 are formed by only one face of the blank B100A. The esthetic appearance of the tray 100A can therefore be improved simply by decorating one face (the first face S10 in this example) of the blank B100A with printing or the like, and this is also advantageous from a cost perspective. In addition, when only the first face S10 serves as a decorative face, the bottom face of the recess 10 also constitutes a decorative face in the tray 100A according to Embodiment 2, therefore achieving a better esthetic than with the tray 100 according to Embodiment 1.

Moreover, a package such as shown in fig. 11 may also be constructed by using the tray 100A according to Embodiment 2. That is to say, the tray 100A may be used in place of the tray 100 or the front-side tray 200 in the package 1000 shown in fig. 11.

Strength Test

Packages employing a tray were subjected to a drop test in order to confirm the strength of the tray according to the embodiment. The package 1000 according to Embodiment 1 was used as the package subjected to the drop test, and the tray 100 according to Embodiment 1 was used as the tray. In the drop test, the presence or absence of deformation of the tray was confirmed when the package was dropped onto a concrete floor from a height of 1 [m]. The faces oriented vertically downward when the package was dropped (i.e., the faces striking the ground; referred to below as dropped faces) were the lower face, upper face, right side face and left side face of the package. Furthermore, a comparison was made for when the basis weight of the blank constituting the material of the tray was 350 [gsm] and 400 [gsm]. Fig. 19 is a table showing results of the drop test. In the table in fig. 19, "O" indicates that no deformation of the tray was apparent in the drop test, and "Δ" indicates that deformation was apparent. As shown in fig. 19, when the basis weight of the blank was 350 [gsm], deformation of the tray was apparent when the dropped faces were the upper face (when the package was dropped from the upper face) and the lower face, and no deformation was apparent when the dropped faces were the right side face and the left side face. Furthermore, when the basis weight of the blank was 400 [gsm], no deformation was apparent for any of the dropped faces among the upper face, lower face, right side face and left side face.

Other

Preferred embodiments according to the present disclosure were described above, but suitable modifications may be added to those embodiments within a scope that does not depart from the main objective of the technology according to the present disclosure.

The following addenda may also be given in regard to the embodiments above.

(Addendum 1)

A heat-not-burn flavor inhaler tray formed by folding a blank and comprising:

    a recess capable of accommodating the heat-not-burn flavor inhaler;
    a hollow cushioning frame body surrounding the recess; and
    a cushioning space formed inside the cushioning frame body,
    wherein the cushioning frame body is formed so as to fit with and hold the heat-not-burn flavor inhaler accommodated in the recess.

(Addendum 2)

The heat-not-burn flavor inhaler tray as disclosed in addendum 1, wherein an opening portion of the recess is formed so as to follow an outer shape of the heat-not-burn flavor inhaler accommodated in the recess.

(Addendum 3)

The heat-not-burn flavor inhaler tray as disclosed in addendum 1 or 2, further comprising a bottom-face cushioning body forming a cushioning space on a bottom portion of the recess.

(Addendum 4)

The heat-not-burn flavor inhaler tray as disclosed in any of addenda 1 to 3, wherein the cushioning frame body is formed by means of multiple hollow cushioning frame portions including: a first cushioning frame portion and a second cushioning frame portion extending in a first direction; and a third cushioning frame portion and a fourth cushioning frame portion extending in a second direction orthogonal to the first direction.

(Addendum 5)

The heat-not-burn flavor inhaler tray as disclosed in addendum 4, wherein both end portions of each of the first cushioning frame portion and the second cushioning frame portion are inclined so that lengths thereof in the first direction decrease closer toward the recess in the second direction;

both end portions of each of the third cushioning frame portion and the fourth cushioning frame portion are inclined so that lengths thereof in the second direction decrease closer toward the recess in the first direction; one end portion of the third cushioning frame portion is inserted at one end portion of the first cushioning frame portion so as to overlap said one end portion, whereby the third cushioning frame portion and the first cushioning frame portion are joined; another end portion of the third cushioning frame portion is inserted at one end portion of the second cushioning frame portion so as to overlap said one end portion, whereby the third cushioning frame portion and the second cushioning frame portion are joined; one end portion of the fourth cushioning frame portion is inserted at another end portion of the first cushioning frame portion so as to overlap said other end portion, whereby the fourth cushioning frame portion and the first cushioning frame portion are joined; and another end portion of the fourth cushioning frame portion is inserted at another end portion of the second cushioning frame portion so as to overlap said other end portion, whereby the fourth cushioning frame portion and the second cushioning frame portion are joined.

(Addendum 6)

The heat-not-burn flavor inhaler tray as disclosed in addendum 5, wherein the pairs of cushioning frame portions of the cushioning frame body which are joined together are configured so that, when θ1 is an angle of inclination in relation to the first direction of an end portion of a cushioning frame portion that receives an end portion of a counterpart side, and when θ2 is an angle of inclination in relation to the second direction of an end portion of a cushioning frame portion that is inserted at an end portion of a counterpart side, then:

$$30[°] \leq \theta 1 \leq 60[°],$$

$$55[°] \leq \theta 2 \leq 85[°],$$

and

$$90[°] < \theta 1 + \theta 2.$$

(Addendum 7)

The heat-not-burn flavor inhaler tray as disclosed in addendum 5 or 6, wherein both end portions of each of the first cushioning frame portion and the second cushioning frame portion are linearly inclined, and both end portions of each of the third cushioning frame portion and the second cushioning frame portion are curved in an arc shape so as to expand outward.

(Addendum 8)

The heat-not-burn flavor inhaler tray as disclosed in addendum 4, wherein the heat-not-burn flavor inhaler is sandwiched by the pairs of cushioning frame portions of the cushioning frame body which face each other, and a pair of cushioning frame portions which face each other are configured so that a gap therebetween becomes narrower from one side toward another side in a direction of extension of the pair of cushioning frame portions.

(Addendum 9)

The heat-not-burn flavor inhaler tray as disclosed in any of addenda 1 to 8, wherein the basis weight of the blank is 400 [gsm] or greater.

(Addendum 10)

The heat-not-burn flavor inhaler tray as disclosed in any one of addenda 1 to 9, wherein the blank comprises: a square base panel having a first edge and a second edge extending in a first direction, and a third edge and a fourth edge extending in a second direction orthogonal to the first direction; and spacer panels j oined to the edges of the base panel; and the cushioning frame body is formed by folding the spacer panels.

(Addendum 11)

The heat-not-burn flavor inhaler tray as disclosed in addendum 10, wherein the cushioning frame body is formed by multiple hollow cushioning frame portions;

the blank comprises: a first spacer panel joined to the first edge of the base panel, a second spacer panel joined to the second edge, a third spacer panel joined to the third edge, a fourth spacer panel joined to the fourth edge, and a cover panel joined to the fourth spacer panel;
the first spacer panel, the second spacer panel, and the third spacer panel each comprise: an outside tube wall panel which is joined to the base panel and folded so as to stand upright in relation to the base panel, a cover-side tube wall panel which is joined to the outside tube wall panel and folded so as to face the base panel, an inside tube wall panel which is joined to the cover-side tube wall panel and folded so as to face the outside tube wall panel, and a base-side tube wall panel which is joined to the inside tube wall panel and folded so as to face the cover-side tube wall panel and lies over the base panel, tubular cushioning frame portions being formed by folding the above panels;
the fourth spacer panel comprises: a base-side groove wall panel which is folded so as to lie over the base panel, a groove bottom wall panel which is joined to the base-side groove wall panel and folded so as to stand upright in relation to the base panel, and a cover-side groove wall panel which is joined to the groove bottom wall panel and folded so as to face the base-side groove wall panel, a recessed cushioning frame portion being formed by folding the above panels;
the cover panel is folded so as to face the base panel; and
an opening of the recess is formed in either one of the base panel and the cover panel.

(Addendum 12)

The heat-not-burn flavor inhaler tray as disclosed in addendum 11, wherein the opening of the recess is formed in the base panel;

the blank further comprises a fifth spacer panel joined to any of the first spacer panel, the second spacer panel, the third spacer panel and the cover panel; and
the fifth spacer panel is folded so as to face the cover panel and thereby constitutes a bottom face of the recess while also forming a cushioning space with the cover panel.

REFERENCE SIGNS LIST

| | |
|---|---|
| 1 | Cushioning frame portion |
| 10 | Recess |
| 20 | Cushioning frame body |
| 30 | Cushioning space |
| 100 | Heat-not-burn flavor inhaler tray |
| B100 | Blank |

**Claims**

1. A heat-not-burn flavor inhaler tray formed by folding a blank and comprising:

   a recess capable of accommodating the heat-not-burn flavor inhaler;
   a hollow cushioning frame body surrounding the recess; and
   a cushioning space formed inside the cushioning frame body,
   wherein the cushioning frame body is formed so as to fit with and hold the heat-not-burn flavor inhaler accommodated in the recess.

2. The heat-not-burn flavor inhaler tray as claimed in claim 1, wherein an opening portion of the recess is formed so as to follow an outer shape of the heat-not-burn flavor inhaler accommodated in the recess.

3. The heat-not-burn flavor inhaler tray as claimed in claim 1 or 2, further comprising a bottom-face cushioning body forming a cushioning space on a bottom portion of the recess.

4. The heat-not-burn flavor inhaler tray as claimed in any one of claims 1 to 3, wherein the cushioning frame body is formed by means of multiple hollow cushioning frame portions including: a first cushioning frame portion and a second cushioning frame portion extending in a first direction; and a third cushioning frame portion and a fourth cushioning frame portion extending in a second direction orthogonal to the first direction.

5. The heat-not-burn flavor inhaler tray as claimed in claim 4, wherein both end portions of each of the first cushioning frame portion and the second cushioning frame portion are inclined so that lengths thereof in the first direction decrease closer toward the recess in the second direction;

both end portions of each of the third cushioning frame portion and the fourth cushioning frame portion are inclined so that lengths thereof in the second direction decrease closer toward the recess in the first direction;
one end portion of the third cushioning frame portion is inserted at one end portion of the first cushioning frame portion so as to overlap said one end portion, whereby the third cushioning frame portion and the first cushioning frame portion are joined;
another end portion of the third cushioning frame portion is inserted at one end portion of the second cushioning frame portion so as to overlap said one end portion, whereby the third cushioning frame portion and the second cushioning frame portion are joined;
one end portion of the fourth cushioning frame portion is inserted at another end portion of the first cushioning frame portion so as to overlap said other end portion, whereby the fourth cushioning frame portion and the first cushioning frame portion are joined; and
another end portion of the fourth cushioning frame portion is inserted at another end portion of the second cushioning frame portion so as to overlap said other end portion, whereby the fourth cushioning frame portion and the second cushioning frame portion are joined.

6. The heat-not-burn flavor inhaler tray as claimed in claim 5, wherein the pairs of cushioning frame portions of the cushioning frame body which are joined together are configured so that, when $\theta 1$ is an angle of inclination in relation to the first direction of an end portion of a cushioning frame portion that receives an end portion of a counterpart side, and when $\theta 2$ is an angle of inclination in relation to the second direction of an end portion of a cushioning frame portion that is inserted at an end portion of a counterpart side, then:

$$30[°] \leq \theta 1 \leq 60[°],$$

$$55[°] \leq \theta 2 \leq 85[°],$$

and

$$90[°] < \theta 1 + \theta 2.$$

7. The heat-not-burn flavor inhaler tray as claimed in claim 5 or 6, wherein both end portions of each of the first cushioning frame portion and the second cushioning frame portion are linearly inclined, and both end portions of each of the third cushioning frame portion and the second cushioning frame portion are curved in an arc shape so as to expand outward.

8. The heat-not-burn flavor inhaler tray as claimed in claim 4, wherein the heat-not-burn flavor inhaler is sandwiched by the pairs of cushioning frame portions of the cushioning frame body which face each other, and a pair of cushioning frame portions which face each other are configured so that a gap therebetween becomes narrower from one side toward another side in a direction of extension of the pair of cushioning frame portions.

9. The heat-not-burn flavor inhaler tray as claimed in any one of claims 1 to 8, wherein the basis weight of the blank is 400 [gsm] or greater.

10. The heat-not-burn flavor inhaler tray as claimed in any one of claims 1 to 9, wherein the blank comprises: a square base panel having a first edge and a second edge extending in a first direction, and a third edge and

a fourth edge extending in a second direction orthogonal to the first direction; and spacer panels joined to the edges of the base panel; and the cushioning frame body is formed by folding the spacer panels.

11. The heat-not-burn flavor inhaler tray as claimed in claim 10, wherein the cushioning frame body is formed by multiple hollow cushioning frame portions;

the blank comprises: a first spacer panel joined to the first edge of the base panel, a second spacer panel joined to the second edge, a third spacer panel joined to the third edge, a fourth spacer panel joined to the fourth edge, and a cover panel joined to the fourth spacer panel; the first spacer panel, the second spacer panel, and the third spacer panel each comprise: an outside tube wall panel which is joined to the base panel and folded so as to stand upright in relation to the base panel, a cover-side tube wall panel which is joined to the outside tube wall panel and folded so as to face the base panel, an inside tube wall panel which is joined to the cover-side tube wall panel and folded so as to face the outside tube wall panel, and a base-side tube wall panel which is joined to the inside tube wall panel and folded so as to face the cover-side tube wall panel and lies over the base panel, tubular cushioning frame portions being formed by folding the above panels;

the fourth spacer panel comprises: a base-side groove wall panel which is folded so as to lie over the base panel, a groove bottom wall panel which is joined to the base-side groove wall panel and folded so as to stand upright in relation to the base panel, and a cover-side groove wall panel which is joined to the groove bottom wall panel and folded so as to face the base-side groove wall panel, a recessed cushioning frame portion being formed by folding the above panels;

the cover panel is folded so as to face the base panel; and

an opening of the recess is formed in either one of the base panel and the cover panel.

12. The heat-not-burn flavor inhaler tray as claimed in claim 11, wherein the opening of the recess is formed in the base panel;

the blank further comprises a fifth spacer panel joined to any of the first spacer panel, the second spacer panel, the third spacer panel and the cover panel; and

the fifth spacer panel is folded so as to face the cover panel and thereby constitutes a bottom face of the recess while also forming a cushioning space with the cover panel.

[Fig. 1]

[Fig. 2]

[Fig. 3]

[Fig. 4]

[Fig. 5]

EP 4 610 190 A1

[Fig. 6]

[Fig. 7]

[Fig. 8]

[Fig. 9]

[Fig. 10]

(A)

(B)

[Fig. 11]

[Fig. 12]

[Fig. 13]

[Fig. 14]

[Fig. 15]

[Fig. 16]

[Fig. 17]

[Fig. 18]

B100A

[Fig. 19]

| Dropped Face | Basis Weight [gsm] | |
|---|---|---|
| | 350 | 400 |
| Upper face | △ | ○ |
| Lower face | △ | ○ |
| Right side face | ○ | ○ |
| Left side face | ○ | ○ |

## INTERNATIONAL SEARCH REPORT

| International application No. |
|---|
| **PCT/JP2022/039445** |

| A. CLASSIFICATION OF SUBJECT MATTER |
|---|
| **B65D 81/02**(2006.01)i; **B65D 5/50**(2006.01)i<br>FI:  B65D81/02 200; B65D5/50 E |
| According to International Patent Classification (IPC) or to both national classification and IPC |

| B. FIELDS SEARCHED |
|---|
| Minimum documentation searched (classification system followed by classification symbols)<br>  B65D81/02; B65D5/50 |
| Documentation searched other than minimum documentation to the extent that such documents are included in the fields searched<br>  Published examined utility model applications of Japan 1922-1996<br>  Published unexamined utility model applications of Japan 1971-2022<br>  Registered utility model specifications of Japan 1996-2022<br>  Published registered utility model applications of Japan 1994-2022 |
| Electronic data base consulted during the international search (name of data base and, where practicable, search terms used) |

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | JP 3037809 U (SHOWA CARTON CO., LTD.) 27 May 1997 (1997-05-27)<br>  paragraph [0017], fig. 1-4 | 1-4, 9-10 |
| A | | 5-8, 11-12 |
| Y | JP 2006-240702 A (FUNAI ELECTRIC CO LTD) 14 September 2006 (2006-09-14)<br>  paragraph [0031], fig. 1-2 | 1-4, 9-10 |
| Y | JP 2007-91331 A (KYOCERA MITA CORP) 12 April 2007 (2007-04-12)<br>  paragraph [0021], fig. 3-4, 5(b) | 3-4, 9-10 |
| Y | JP 11-171168 A (SHARP CORP) 29 June 1999 (1999-06-29)<br>  fig. 2-3 | 10 |
| A | JP 2009-262951 A (SHARP CORP) 12 November 2009 (2009-11-12) | 11-12 |
| A | JP 2012-214247 A (RENGO CO LTD) 08 November 2012 (2012-11-08) | 11-12 |

☐ Further documents are listed in the continuation of Box C.  ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 November 2022** | **29 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT

Information on patent family members

International application No.

**PCT/JP2022/039445**

| Patent document cited in search report | | | Publication date (day/month/year) | Patent family member(s) | Publication date (day/month/year) |
|---|---|---|---|---|---|
| JP | 3037809 | U | 27 May 1997 | (Family: none) | |
| JP | 2006-240702 | A | 14 September 2006 | (Family: none) | |
| JP | 2007-91331 | A | 12 April 2007 | (Family: none) | |
| JP | 11-171168 | A | 29 June 1999 | (Family: none) | |
| JP | 2009-262951 | A | 12 November 2009 | (Family: none) | |
| JP | 2012-214247 | A | 08 November 2012 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013049434 A
- JP 2009096520 A
- JP 3200836 U
- JP 2005014965 A